# EUROPEAN PATENT APPLICATION

(11) **EP 3 782 702 A1**
(43) Date of publication of application: **24.02.2021**
(21) Application number: 19192793.8
(22) Date of filing: 21.08.2019
(51) Int. Cl.: A61P 31/00, A61P 35/00, C07D 233/64, C07D 263/32, C07D 277/28, C07D 413/04, C07D 417/04

(54) **COMPOUNDS AND USE THEREOF FOR THE TREATMENT OF INFECTIOUS DISEASES AND CANCER**

(71) Applicant: AC BioScience SA, 1024 Ecublens (CH)
(72) Inventor: AUCLAIR, Christian, 78730 Saint Arnoult en Yvelines (FR); IVES, Annette, 1132 Lully (CH)
(74) Representative: KATZAROV S.A.

(57) **Abstract**

The invention provides the imidazole, oxazole and thiazole compounds and use thereof in methods for treating a disease or a disorder, such as infectious diseases and cancer, wherein inhibition of sphingosine-1-phosphate lyase is beneficial to treat the disease or the disorder.

## Description

### FIELD OF THE INVENTION

The invention provides the imidazole, oxazole and thiazole compounds and use thereof in methods for treating a disease or a disorder, such as infectious diseases and cancer, wherein inhibition of sphingosine-1-phosphate lyase is beneficial to treat the disease or the disorder.

### BACKGROUND OF THE INVENTION

Sphingosine-1-phosphate (S1P) is a lipid metabolite that is produced from ceramide and other higher order sphingolipids by sphingosine kinase (SphK1 and SphK2). SIP levels are tightly controlled through production via SphKs, recycling back into sphingosine by sphingosine phosphatases (SGPP1 and 2) or lipid phosphate phosphatases (LPP1 and 2) and irreversible degradation through sphingosine-1-phosphate lyase (S1PL; also known as SGPL1 in mammals) into phosphoethanolamine (pEtN) and 2-hexadecanal. Sphingosine-1-phosphate lyase is a vitamin B6-dependent enzyme localized in the membrane of the endoplasmic reticulum. Like many products of sphingomyelin, it exhibits a wide range of biological activities. S1P enhances cell growth and promotes proliferation, differentiation, and survival in different cell systems and can inhibit the normal apoptotic response to a variety of stimuli. Many of the activities of S1P are mediated through five closely related G-protein-coupled receptors family (S1PR) which play a crucial role in sphingolipid signalling. Each of these receptors appears to be tissue specific and to have demonstrated roles in the regulation of cell proliferation and survival in mammalian cells and in various cancer types. S1P increases intracellular calcium levels by both the liberation of calcium from internal stores and through activation of a Gi protein-coupled receptor resulting in the opening of a cell surface calcium channel. Among number of biological effects, S1P signaling is a potent effector of vascular cell adhesion and motility that are important for blood vessel formation. Most data support the concept that S1P signaling via specific S1P receptors, mainly S1PR1, and to a lesser extent S1PR3, orchestrates key events along the continuum of blood vessel formation. Substantial evidence exists for S1P serving as a pro-angiogenic factor as well as an inducer of vascular maturation. A key aspect of the effect of S1P in angiogenesis relates to its ability promote intercellular interactions between endothelial cells and smooth muscle cells that are necessary for the formation of adherent junctions and subsequent stabilization of newly formed blood vessels. In tumor microenvironment this S1P property leads to a vasculature normalization improving the immunological status of the oxygen-enriched tumor and improving responses to conventional anticancer therapies including radiotherapy and chemotherapy. Taking together, it can be concluded that a high concentration of S1P in blood and tissues may have beneficial effects in certain pathological circumstances.

In addition to its role in angiogenesis, S1P and its metabolites 2-hexadecanal and pEtN have clear ability to impact the immune system. Yet, the exact roles are cell type dependent, related to the mechanisms of production and the specific pathobiology of the disease. Many of the effects of S1P are through the modulation of immune cell survival, endocytosis, antigen presentation, redox-dependent stress responses, and the modulation of cell signaling pathways such as Nuclear Factor kappa B (NFkB). Studies have also elucidated roles for 2-hexadecanal and pEtN in stress responses, cell death and cytoskeletal organization.

Mice deficient for S1PL die shortly after birth and have compromised immune systems as demonstrated by their severe inflammatory phenotypes. Similarly mice deficient for both SpHK1 and SpHK2 are embryonically lethal.

It is known that 2-acetyl-4-tetrahydroxybutylimidazole (THI) inhibits S1P lyase activity when administered to mice (Schwab, S. et al., Science 309:1735-1739; 2005). It appeared therefore possible to treat some diseases by inhibiting the enzyme S1P lyase (S1PL).

Pathogenic infections, such as bacterial infections, can be addressed by S1PL modulation of the host and/or the pathogen. Functional S1PLs has been identified in several bacteria, such as *Legionella pneumoniae*, and *Burkholderia spp*., which cause pulmonary infections of Legionnaire's disease and Melioidosis, respectively.

Modulation of S1P mediated biological actions both at the intracellular and extracellular level can improve pathogen killing within infected immune cells, including the macrophage. For example, in *Leishmania donovani*-infected macrophages, administration of S1P improved parasite clearance while abrogation of SphK activity that produces S1P, and the inhibition of S1PR2 and S1PR2 ligation results in elevated parasite burden within the cells and a modulation of the host immune response.

Additionally, experimental evidence using *leishmania* major deficient for S1PL demonstrate that this molecule can affect the parasite infectivity and delays disease development in mice. This is due to the fact that for this specific parasite they require host-derived sphingolipids for the formation of cell membrane components. Noticeably, also *L. major* parasites deficient for the SphK (required for S1P production) were resistant to infection in a susceptible mouse model. Similarly, *Burkholderia spp.* and *Legionella pneumophilia* have secreted forms of S1PL. *In vivo* infection models using S1PL-deficient bacteria display reduced infection levels and pathogenesis. *In vitro* macrophage studies confirmed that a functional S1PL produced by these bacteria mediate intracellular persistence and modulation of host immune responses to promote survival.

Putative Sphingosine-1-phosphate lyase genes have been found with in many different pathogenic organisms including helminths (e.g.: Schistosoma Masoni), other kinetoplasts (e.g.: Trypanosoma cruzi and Trypanosoma brucei), and species of pathogenic fungi (e.g.: Candida albicans).

Currently leishmaniasis and other neglected tropical diseases (NTDS), which includes, amongst others, Chagas disease and Human African Trypanosomiasis-both caused by infection by *Trypanosoma spp* are of public health concern predominantly in low and low-middle income countries. Annual estimates indicate up to 1 million cases of cutaneous leishmaniasis (CL), 90,000 cases of Visceral leishmaniasis (VL), 7 million cases of Chagas disease. For Human African Trypanosomiasis, approximately 10.5 million people have a med/high risk of infection. These diseases, depending on host genetics and the infecting parasite, can be fatal, if left untreated. Although some forms of CL may be a self-limiting, there is the possibility for the development of the life-threatening secondary disease of mucocutaneous leishmaniasis (MCL) and the development on non-healing progressive CL lesions that require treatment. For example, even though CL may ultimately be resolved it is often with significant amount of scarring.

Thus modulation of sphingosine-1-phosphate (S1P) activity for the treatment of related diseases or disorders, such as infectious diseases and cancer, is still an unmet need. More in particularly, potent and effective S1P lyase inhibitors that modulate the levels SIP and its metabolites 2-hexadecanal and pEtN are needed.

### SUMMARY OF THE INVENTION

An aspect of the present invention provides a compound of Formula I 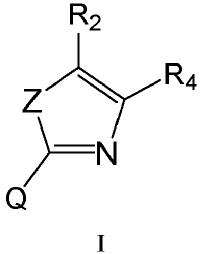 wherein
Z is selected from the group comprising O, S, NH;
Q is 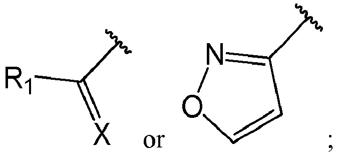
X is O or NR₃;
R₁ is selected from the group comprising OR_{A}, NHOH, hydrogen, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl;
R₂ is selected from the group comprising OR_{B}, C(O)OR_{B}, hydrogen, halogen, nitrile, optionally substituted hydroxyalkyl, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl;
R₃ is selected from the group comprising OR_{C}, N(R_{C})₂, NHC(O)R_{C}, NHSO₂R_{C}, hydrogen;
R₄ is selected from the group comprising OR_{D}, OC(O)R_{D}, N(R_{E})₂, hydrogen, halogen, optionally substituted hydroxyalkyl, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl; and
each of R_{A}, R_{B}, R_{C}, R_{D}, and R_{E} is independently hydrogen or optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl.

Another aspect of the present invention provides a pharmaceutical composition comprising the compound of the invention and pharmaceutically acceptable excipients and/or carriers.

Another aspect of the present invention provides the compound of the invention for use in a method of increasing the amount of S1P in a subject.

Another aspect of the present invention provides the compound of the invention for use in a method of preventing and/or treating infectious diseases in a subject, wherein the infectious diseases are caused by pathogenic microorganisms selected from the group comprising bacteria, viruses, fungi and parasites.

Another aspect of the present invention provides the compound of the invention for use in a method for improving the efficacy of a chemotherapy and/or a radiotherapy, wherein a therapeutically effective amount of the compound of the invention is administered to a subject prior to or during the chemotherapy and/or the radiotherapy.

Another aspect of the present invention provides the compound of the invention for use in a method of treatment of cancer, wherein a therapeutically effective amount of the compound of the invention is administered to a subject prior to or during the chemotherapy and/or the radiotherapy.

### DETAILED DESCRIPTION OF THE INVENTION

All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. Also as used in the specification and claims, the language "comprising" can include analogous embodiments described in terms of "consisting of "and/or "consisting essentially of'.

As used in the specification and claims, the singular form "a", "an" and "the" include plural references unless the context clearly dictates otherwise.

As used in the specification and claims, the term "and/or" used in a phrase such as "A and/or B" herein is intended to include "A and B", "A or B", "A", and "B".

The term "alkyl" means a straight chain, branched and/or cyclic ("cycloalkyl") hydrocarbon having from 1 to 20 or 1 to 10 or 1 to 4 carbon atoms. Alkyl moieties having from 1 to 4 carbons are referred to as "lower alkyl". Examples of alkyl groups include, but are not limited to, methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl and dodecyl. Cycloalkyl moieties may be monocyclic or multicyclic, and examples include cyclopropyl, cyclobutyl, cyclopentyl, cyclohexyl, and adamantyl. Additional examples of alkyl moieties have linear, branched and/or cyclic portions (e.g., 1-ethyl-4-methyl-cyclohexyl). The term "alkyl" includes saturated hydrocarbons as well as alkenyl and alkynyl moieties.

The term "alkenyl" means a straight chain, branched and/or cyclic hydrocarbon having from 2 to 20 (e.g., 2 to 10 or 2 to 6) carbon atoms, and including at least one carbon-carbon double bond. Representative alkenyl moieties include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl and 3-decenyl.

The term "alkylaryl" or "alkyl-aryl" means an alkyl moiety bound to an aryl moiety.

The term "alkylheteroaryl" or "alkyl-heteroaryl" means an alkyl moiety bound to a heteroaryl moiety.

The term "alkylheterocycle" or "alkyl-heterocycle" means an alkyl moiety bound to a heterocycle moiety.

The term "alkynyl" means a straight chain, branched or cyclic hydrocarbon having from 2 to 20 or 2 to 20 or 2 to 6 carbon atoms, and including at least one carbon-carbon triple bond. Representative alkynyl moieties include acetylenyl, propynyl, 1-butynyl, 2-butynyl, 1-pentynyl, 2-pentynyl, 3-methyl-1-butynyl, 4-pentynyl, 1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl and 9-decynyl.

The term "alkoxy" means an -O-alkyl group. Examples of alkoxy groups include, but are not limited to, -OCH₃, -OCH₂CH₃, -O(CH₂)₂CH₃, -O(CH₂)₃CH₃, -O(CH₂)₄CH₃, and -O(CH₂)₅CH₃.

The term "aryl" means an aromatic ring or an aromatic or partially aromatic ring system composed of carbon and hydrogen atoms. An aryl moiety may comprise multiple rings bound or fused together. Examples of aryl moieties include, but are not limited to, anthracenyl, azulenyl, biphenyl, fluorenyl, indan, indenyl, naphthyl, phenanthrenyl, phenyl, 1,2,3,4-tetrahydro-naphthalene, and tolyl.

The term "arylalkyl" or "aryl-alkyl" means an aryl moiety bound to an alkyl moiety.

The terms "halogen" and "halo" encompass fluorine, chlorine, bromine, and iodine.

The term "heteroalkyl" refers to an alkyl moiety (linear, branched or cyclic) in which at least one of its carbon atoms has been replaced with a heteroatom (such as N, O or S).

The term "heteroaryl" means an aryl moiety wherein at least one of its carbon atoms has been replaced with a heteroatom (such as N, O or S). Examples include, but are not limited to, acridinyl, benzimidazolyl, benzofuranyl, benzoisothiazolyl, benzoisoxazolyl, benzoquinazolinyl, benzothiazolyl, benzoxazolyl, furyl, imidazolyl, indolyl, isothiazolyl, isoxazolyl, oxadiazolyl, oxazolyl, phthalazinyl, pyrazinyl, pyrazolyl, pyridazinyl, pyridyl, pyrimidinyl, pyrimidyl, pyrrolyl, quinazolinyl, quinolinyl, tetrazolyl, thiazolyl, and triazinyl.

The term "heteroarylalkyl" or "heteroaryl-alkyl" means a heteroaryl moiety bound to an alkyl moiety.

The term "heterocycle" refers to an aromatic, partially aromatic or non-aromatic monocyclic or polycyclic ring or ring system comprised of carbon, hydrogen and at least one heteroatom (e.g., N, O or S). A heterocycle may comprise multiple (i.e., two or more) rings fused or bound together. Heterocycles include heteroaryls. Examples include, but are not limited to, benzo[1,3]dioxolyl, 2,3-dihydro-benzo[1,4]dioxinyl, cinnolinyl, furanyl, hydantoinyl, morpholinyl, oxetanyl, oxiranyl, piperazinyl, piperidinyl, pyrrolidinonyl, pyrrolidinyl, tetrahydrofuranyl, tetrahydropyranyl, tetrahydropyridinyl, tetrahydropyrimidinyl, tetrahydrothiophenyl, tetrahydrothiopyranyl and valerolactamyl.

The term "heterocyclealkyl" or "heterocycle-alkyl" refers to a heterocycle moiety bound to an alkyl moiety.

The term "heterocycloalkyl" refers to a non-aromatic heterocycle.

The term "heterocycloalkylalkyl" or "heterocycloalkyl-alkyl" refers to a heterocycloalkyl moiety bound to an alkyl moiety.

The term "substituted," when used to describe a chemical structure or moiety, refers to a derivative of that structure or moiety wherein one or more of its hydrogen atoms is substituted with a chemical moiety or functional group such as, but not limited to, alcohol, aldehylde, alkoxy, alkanoyloxy, alkoxycarbonyl, alkenyl, alkyl (e.g., methyl, ethyl, propyl, t-butyl), alkynyl, alkylcarbonyloxy (-OC(O)alkyl), amide (-C(O)NH-alkyl- or -alkylNHC(O)alkyl), amidinyl (-C(NH)NH-alkyl or -C(NR)NH2), amine (primary, secondary and tertiary such as alkylamino, arylamino, arylalkylamino), aroyl, aryl, aryloxy, azo, carbamoyl (-NHC(O)O-alkyl- or -OC(O)NH-alkyl), carbamyl (for example CONH₂, as well as CONH-alkyl, CONH-aryl, and CONH-arylalkyl), carbonyl, carboxyl, carboxylic acid, carboxylic acid anhydride, carboxylic acid chloride, cyano, ester, epoxide, ether (e.g., methoxy, ethoxy), guanidino, halo, haloalkyl (for example -CCl₃, -CF₃, -C(CF₃)₃), heteroalkyl, hemiacetal, imine (primary and secondary), isocyanate, isothiocyanate, ketone, nitrile, nitro, oxo, phosphodiester, sulfide, sulfonamido (e.g., SO₂NH₂), sulfone, sulfonyl (including alkylsulfonyl, arylsulfonyl and arylalkylsulfonyl), sulfoxide, thiol (e.g., sulfhydryl, thioether) and urea (-NHCONH-alkyl-).

Some compounds of the present invention can exist in a tautomeric form which is also intended to be encompassed within the scope of the present invention. "Tautomers" refers to compounds whose structures differ markedly in the arrangement of atoms, but which exist in easy and rapid equilibrium. It is to be understood that compounds of present invention may be depicted as different tautomers. It should also be understood that when compounds have tautomeric forms, all tautomeric forms are intended to be within the scope of the present invention, and the naming of the compounds does not exclude any tautomeric form. Tautomers exist as mixtures of a tautomeric set in solution. In solid form, usually one tautomer predominates. Even though one tautomer may be described, the present invention includes all tautomers of the compounds disclosed herein. A tautomer is one of two or more structural isomers that exist in equilibrium and are readily converted from one isomeric form to another. This reaction results in the formal migration of a hydrogen atom accompanied by a shift of adjacent conjugated double bonds. In solutions where tautomerization is possible, a chemical equilibrium of the tautomers can be reached. The exact ratio of the tautomers depends on several factors, including temperature, solvent, and pH. The concept of tautomers that are interconvertible by tautomerizations is called tautomerism. Tautomerizations are catalyzed by: Base: 1. deprotonation; 2. formation of a delocalized anion (e.g., an enolate); 3. protonation at a different position of the anion; Acid: 1. protonation; 2. formation of a delocalized cation; 3. deprotonation at a different position adjacent to the cation.

As used herein the terms "subject" and "patient" are well-recognized in the art, and, are used herein to refer to a mammal, including, for example, humans, animals, domestic pets, livestock and other farm animals; the most preferably a mammal is a human. In some embodiments, the subject is a subject in need of treatment or a subject being infected by pathogens and/or parasites. The term does not denote a particular age or sex. Thus, adult and newborn subjects, whether male or female, are intended to be covered.

As used herein the term "pharmaceutically acceptable excipients and/or carriers" means that the compositions or components thereof so described are suitable for suitable for administration to subject (patient) body without undue toxicity, incompatibility, instability, irritability, allergic response, and the like.

The term "treat" and its grammatical variants (for example "to treat," "treating," and "treatment") refer to administration of an active pharmaceutical ingredient to a subject (patient), such as the compound of the invention, with the purpose of inhibiting a disease or a disorder (such as infectious disease or cancer) in a subject (patient). Treating the disease or the disorder includes ameliorating at least one symptom, reducing severity, impeding progress, and/or curing the subject (patient) of the disease or the disorder. In the present context, treatment entails oral, parenteral, mucosal and/or topical administration of the compounds of the invention or the pharmaceutical composition of the invention to a subject (patient).

As used herein, the term "preventing" refers to prophylactic steps taken to reduce the likelihood of a subject from contracting or suffering from a particular disease or disorder (such as infectious disease or cancer). The likelihood of the disease or the disorder occurring in the subject need not be reduced to zero for the preventing to occur; rather, if the steps reduce the risk of a disease or a disorder across a population, then the steps prevent the disease or the disorder within the scope and meaning herein.

As used herein the term "administration" or "administering" to a subject refers to any means of introducing the compound of the invention or the pharmaceutical composition of the invention onto and/or into the subject (patient) body or a portion thereof. According to an embodiment for administration to animals, the compounds and pharmaceutical composition of the invention may be provided through the food administered to the subject.

The term "therapeutically effective amount," as used herein, refers to any amount of a compound of the invention that will cause inhibition of a disease or a disorder (such as infectious disease or cancer) in a subject and thus reduction of symptoms, disappearance of the symptoms or relief from symptoms related to a disease or a disorder (such as infectious disease or cancer), when applied, either once, or repeatedly over time. Therapeutically effective amounts can be readily determined by persons skilled in the art using routine experimentation and using tests and measures commonly employed in the art, or can be based upon the subjective response of patients undergoing treatment.

The term "prophylactically effective amount," as used herein, is intended to include the amount of a compound of the invention that, when administered to a subject who does not yet experience or display symptoms of a disease or a disorder, such as infectious disease), but who may be predisposed to the disease or the disorder, is sufficient to prevent or ameliorate the disease or the disorder or one or more symptoms of the disease or the disorder. Ameliorating the disease or the disorder includes slowing the course of the disease or the disorder or reducing the severity of later-developing disease or disorder. The "prophylactically effective amount" can be readily determined by persons skilled in the art using routine experimentation and using tests and measures commonly employed in the art, or can be based upon the subjective response of patients undergoing treatment.

The term "neoadjuvant", as used herein, refers to administration of the compound of the invention prior to or during the main chemotherapeutical and/or radiotherapeutical treatment, with the intent of boosting the efficacy or increasing the effectiveness of the chemotherapy and/or radiotherapy.

The present invention provides a family of compounds or tautomers thereof, that can be used as inhibitors of S1P lyase. The present invention also includes pharmaceutically acceptable salts of said compounds and tautomers.

An aspect of the present invention provides a compound of Formula I 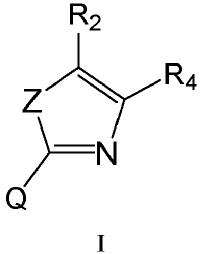 wherein
Z is selected from the group comprising O, S, NH, preferably Z is O or S;
Q is 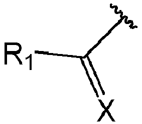 or optionally substituted heterocycle; preferably Q is 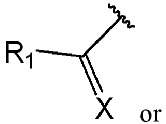 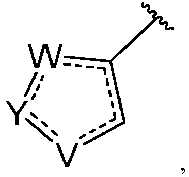 more preferably Q is 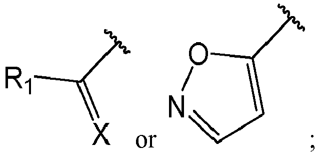
X is O or NR₃;
each of W, Y, V is independently selected from the group comprising CH₂, CH, N, NH, O or S.
R₁ is selected from the group comprising OR_{A}, NHOH, hydrogen, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl; preferably R₁ is C₁-C₅ alkyl; more preferably R₁ is -CH₃;
R₂ is selected from the group comprising OR_{B}, C(O)OR_{B}, hydrogen, halogen, nitrile, optionally substituted hydroxyalkyl, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl; preferably, R₂ is hydrogen or -(CH₂)ₙ-OH, wherein n is 1 to 5, preferably n is 1;
R₃ is selected from the group comprising OR_{C}, N(R_{C})₂, NHC(O)R_{C}, NHSO₂R_{C}, hydrogen; preferably, R₃ is -OH
R₄ is selected from the group comprising OR_{D}, OC(O)R_{D}, N(R_{E})₂, hydrogen, halogen, optionally substituted hydroxyalkyl, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl; preferably R₄ is hydrogen or -(CH₂)ₙ-OH, wherein n is 1 to 5, preferably n is 1;
each of R_{A}, R_{B}, R_{C}, R_{D}, and R_{E} is independently selected from the group comprising hydrogen or optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl.

In some embodiments, the compound of the invention is selected from the group comprising

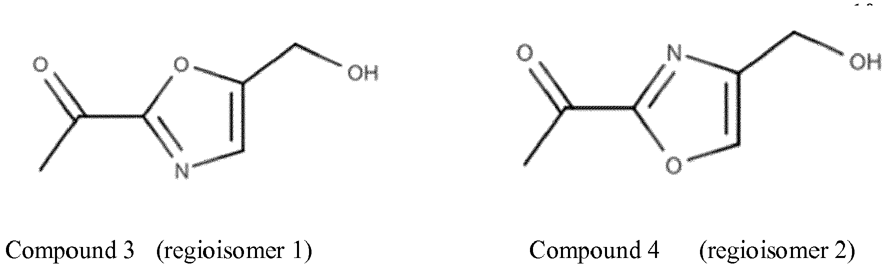

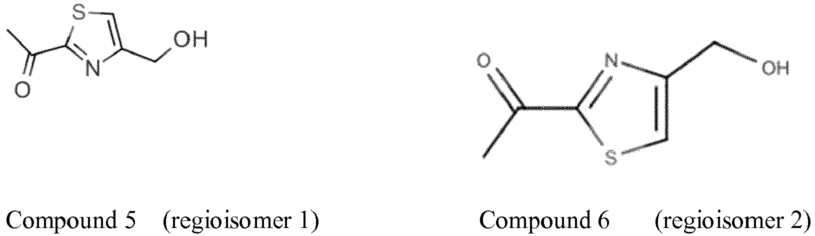

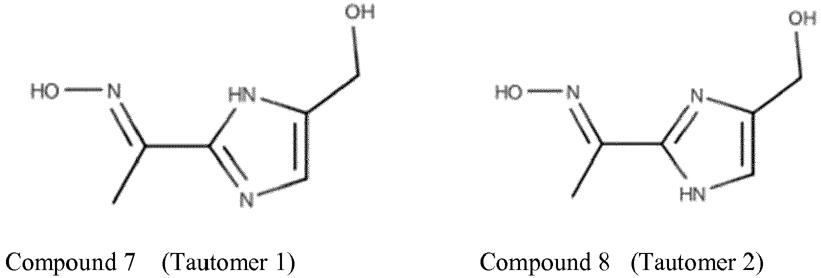

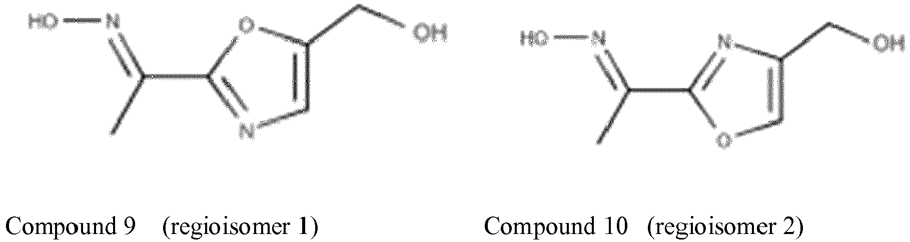

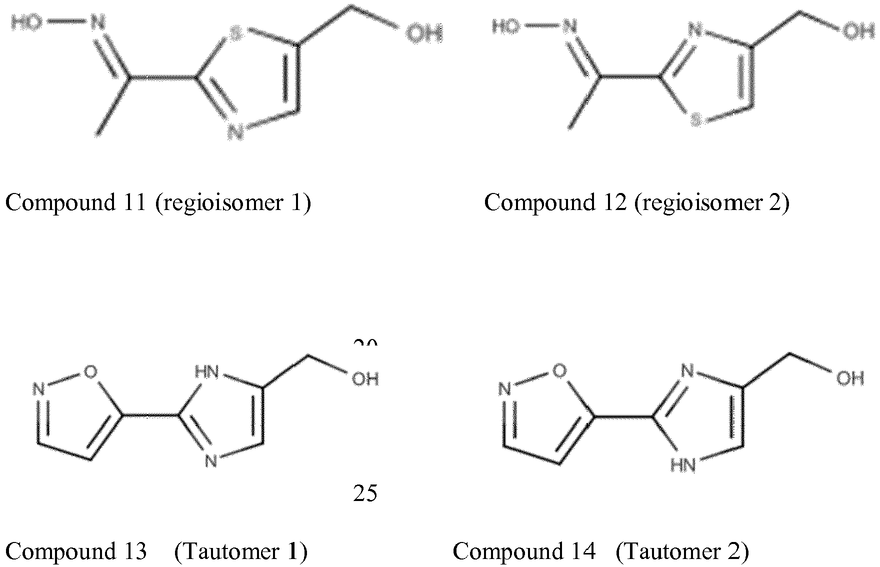

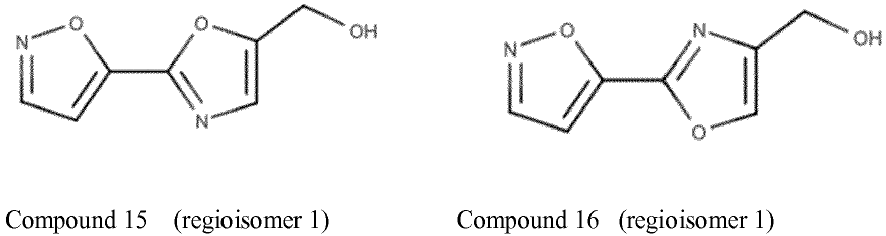

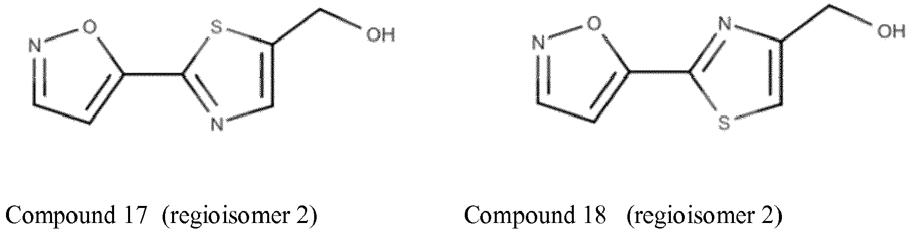

The compounds of the invention can be prepared by methods known in the art.

The compounds of formula I or tautomers thereof, or pharmaceutically acceptable salts of said compounds or tautomers disclosed herein can have asymmetric centres. The compounds of Formula I or tautomers thereof, or pharmaceutically acceptable salts of said compounds or tautomers of the present invention containing an asymmetrically substituted atom can be isolated in optically active or racemic forms. It is well known in the art how to prepare optically active forms, such as by resolution of racemic forms or by synthesis from optically active starting materials. Cis and trans geometric isomers of the compounds of Formula I or tautomers thereof, or pharmaceutically acceptable salts of said compounds or tautomers of the present invention are described and can be isolated as a mixture of isomers or as separate isomeric forms. All chiral, diastereomeric, racemic, and geometric isomeric forms of a structure are intended, unless specific stereochemistry or isomeric form is specifically indicated. All processes used to prepare compounds of Formula I or tautomers thereof, or pharmaceutically acceptable salts of said compounds or tautomers of the present invention and intermediates made herein are considered to be part of the present invention. All tautomers of shown or described compounds are also considered to be part of the present invention.

Specifically, the compounds of Formula I can contain one or more asymmetric centres and can thus occur as racemates and racemic mixtures, single enantiomers, diastereomeric mixtures and individual diastereomers. The present invention is meant to comprehend all such isomeric forms of the compounds of Formula I. The compounds of Formula I may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration. Alternatively, any stereoisomer of a compound of the general structural Formula I may be obtained by stereospecific synthesis using optically pure starting materials or reagents of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base.

The diasteromeric derivatives (see Table 1) may then be converted to the pure enantiomers by cleavage of the added chiral residue. The racemic mixture of the compounds can also be separated directly by chromatographic methods utilizing chiral stationary phases, which methods are well known in the art.

**Table 1. Tautomers and regioisomers of the compounds of the invention**

| Compound No. | Tautomer (Tau.) | Regioisomer (Reg.) | Ring |
|---|---|---|---|
| 3 - ACB1903 | | 1 | oxazole |
| 4 - ACB1904 | | 2 | oxazole |
| 5 - ACB1905 | | 1 | thiazole |
| 6 - ACB1906 | | 2 | thiazole |
| 7 - ACB1907 | 1 | | imidazole |
| 8 - ACB1908 | 2 | | imidazole |
| 9 - ACB1909 | | 1 | oxazole |
| 10 - ACB1910 | | 2 | oxazole |
| 11 - ACB1911 | | 1 | thiazole |
| 12 - ACB1912 | | 2 | thiazole |
| 13 - ACB1913 | 1 | | imidazole |
| 14 - ACB1914 | 2 | | imidazole |
| 15 - ACB1915 | | 1 | oxazole |
| 16-ACB1916 | | 2 | oxazole |
| 17 - ACB1917 | | 1 | thiazole |
| 18 - ACB1918 | | 2 | thiazole |

The compounds of formula (I) or tautomers thereof, or pharmaceutically acceptable salts of said compounds or tautomers disclosed herein have at least one side chain that is typically an aliphatic primary alcohol such as -(CH₂)ₙ-OH, were n is preferably 1, that can be phosphorylated by PKA and/or by other enzymes, such as pyridoxal kinase, during metabolization of the compounds of the invention in the human body and/or in pathogenic microorganisms.

Another aspect of the present invention provides a pharmaceutical composition comprising the compound of the invention and pharmaceutically acceptable excipients and/or carriers.

Certain pharmaceutical compositions of the invention are single unit dosage forms suitable for oral or mucosal (such as nasal, sublingual, vaginal, buccal, or rectal) administration to a patient. Examples of dosage forms include, but are not limited to tablets, caplets, capsules, such as soft elastic gelatine capsules, cachets, troches, lozenges, dispersions, suppositories, powders, solutions, aerosols (such as nasal sprays or inhalers), liquid dosage forms suitable for oral or mucosal administration to a patient, including suspensions (such as aqueous or non-aqueous liquid suspensions, oil-in-water emulsions, or a water-in-oil liquid emulsions), solutions, and elixirs.

The formulation of the pharmaceutical composition of the invention should suit the mode of administration. For example, oral administration requires enteric coatings to protect the compounds of this invention from degradation within the gastrointestinal tract. Similarly, a formulation may contain ingredients that facilitate delivery of the active ingredient(s) to the site of action. For example, compounds may be administered in liposomal formulations, in order to protect them from degradative enzymes, facilitate transport in circulatory system, and effect delivery across cell membranes to intracellular sites.

The pharmaceutical compositions of the invention suitable for oral administration can be presented as discrete dosage forms, such as, but are not limited to, tablets (such as chewable tablets), caplets, capsules, and liquids (such as flavoured syrups). Such dosage forms contain predetermined amounts of active ingredients, and may be prepared by methods of pharmacy well known to those skilled in the art. See, e.g., Remington's Pharmaceutical Sciences, 18th ed., Mack Publishing, Easton Pa. (1990).

Typical oral dosage forms are prepared by combining the compound of the invention in an intimate admixture with at least one excipient according to conventional pharmaceutical compounding techniques. Excipients can take a wide variety of forms depending on the form of preparation desired for administration.

Because of their ease of administration, tablets and capsules represent the most advantageous oral dosage unit forms. If desired, tablets can be coated by standard aqueous or nonaqueous techniques. Such dosage forms can be prepared by conventional methods of pharmacy. In general, pharmaceutical compositions and dosage forms are prepared by uniformly and intimately admixing the compound of the invention with liquid carriers, finely divided solid carriers, or both, and then shaping the product into the desired presentation if necessary. Disintegrants may be incorporated in solid dosage forms to facility rapid dissolution. Lubricants may also be incorporated to facilitate the manufacture of dosage forms (such as tablets). For example pharmaceutically acceptable excipients particularly suitable for use in conjunction with tablets include, for example, inert diluents, such as celluloses, calcium or sodium carbonate, lactose, calcium or sodium phosphate; disintegrating agents, such as cross-linked povidone, maize starch, or alginic acid; binding agents, such as povidone, starch, gelatin or acacia; and lubricating agents, such as magnesium stearate, stearic acid or talc.

Other pharmaceutical compositions of the invention are transdermal and topical dosage forms administered to a patient. Such dosage forms are selected from the group comprising ophthalmic solutions, sprays, aerosols, creams, lotions, ointments, gels, solutions, emulsions, suspensions, or other forms known to a person skilled in the art (see for example Remington's Pharmaceutical Sciences, 16th and 18th eds., Mack Publishing, Easton Pa. (1980 & 1990); and Introduction to Pharmaceutical Dosage Forms, 4th ed., Lea & Febiger, Philadelphia (1985). Transdermal dosage forms include "reservoir type" or "matrix type" patches, which can be applied to the skin and worn for a specific period of time to permit the penetration of a desired amount of the compounds of the invention.

Suitable excipients (e.g., carriers and diluents) and other materials that can be used to provide transdermal, topical, and mucosal dosage forms are well known to a person skilled in the art, and depend on the particular tissue to which a given pharmaceutical composition or dosage form will be applied.

Depending on the specific tissue to be treated, additional components may be used prior to, in conjunction with, or subsequent to treatment with the compounds of the invention. For example, penetration enhancers may be used to assist in delivering compounds of the invention to the tissue.

The pH of a pharmaceutical composition or dosage form, or of the tissue to which the pharmaceutical composition or dosage form is applied, may also be adjusted to improve delivery of one or more compounds of the invention. Similarly, the polarity of a solvent carrier, its ionic strength, or tonicity can be adjusted to improve delivery. Compounds such as stearates may also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

Other pharmaceutical compositions of the invention are parenteral dosage forms administered to patients by various routes including, but not limited to, subcutaneous, intravenous (including bolus injection), intramuscular, and intraarterial. Because their administration typically bypasses patients' natural defenses against contaminants, parenteral dosage forms are specifically sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to a person skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate. The composition, shape, and type of a dosage form of the pharmaceutical composition of the invention will vary depending on its use. For example, a dosage form used in the acute treatment of a disease, such as infectious diseases or cancer, may contain larger amounts of one or more of the compounds of the invention than a dosage form used in the chronic treatment of the same disease.

Another aspect of the present invention provides a method of inhibiting sphingosine-1-phosphate lyase activity in a subject and/or in pathogenic microorganisms selected from the group comprising bacteria, viruses, fungi and parasites, preferably bacteria, fungi and parasites, the method comprising using the compound of the invention. In a specific embodiment, the present invention provides a method of inhibiting sphingosine-1-phosphate lyase activity in a subject, the method comprising using the compound of the invention. Using the compound of the invention includes administering the compound of the invention to the subject. In another specific embodiment, the present invention provides a method of inhibiting sphingosine-1-phosphate lyase activity in pathogenic microorganisms selected from the group comprising bacteria, viruses, fungi and parasites, preferably bacteria, fungi and parasites, the method comprising using the compound of the invention. Using the compound of the invention includes contacting the pathogenic microorganisms with the compound of the invention. In another specific embodiment, the subject can be infected with the pathogenic microorganism and in this context the present invention provides a method of inhibiting sphingosine-1-phosphate lyase activity in a subject and in pathogenic microorganisms selected from the group comprising bacteria, viruses, fungi and parasites, preferably bacteria, fungi and parasites, the method comprising using the compound of the invention. Using the compound of the invention includes administering the compound of the invention to the subject infected with the pathogenic microorganisms.

In a specific embodiment, the present invention provides a use of the compound of the invention for inhibiting S1P lyase in a subject and/or in pathogenic microorganisms selected from the group comprising bacteria, fungi and parasites.

The compounds of the invention are inhibitors of S1P lyase and thus also S1P level enhancing agents and interfere with the action of S1PL in mammalian cells. Aside from elevating S1P level, inhibition of S1P lyase has also influence on other biological effects, such as the products of SIP lyase: 2-hexadecanal and phosphoethanolamine (pEtN) which can influence cellular responses and survival. Thus the compounds of the invention are useful in the treatment, prevention or suppression of diseases and disorders known to be susceptible to variations in the levels of sphingosine-1-phosphate, 2-hexadecanal levels and phosphoethanolamine (pEtN), such as infectious diseases and cancer.

A further aspect of the present invention provides a method for modulating sphingosine-1-phosphate lyase activity, comprising contacting a sphingosine-1-phosphate lyase with an effective amount of a compound of the invention that modulates sphingosine-1-phosphate lyase activity, wherein the step of contacting is performed under conditions and for a time sufficient to allow the compound of the invention and the sphingosine-1-phosphate lyase to interact.

The compounds of the invention increase the amount of S1P, while decreasing the levels of its metabolites. Thus the compounds of the invention would also make use of the immunomodulatory capabilities of S1P, and its metabolites, and boost the development of protective immune responses in the infected host (a subject affected by the infectious diseases).

Another aspect of the present invention provides a method of increasing the amount of SIP in a subject, comprising administering to the subject an effective amount of a compound of the invention.

In a specific embodiment, the present invention provides a compound of the invention for use in a method of increasing the amount of S1P in a subject.

Without being limited by theory and in addition to acting on host cells, including those of the immune system, the compounds of the invention can target and affect pathogen-derived S1PL, which is a virulence factor for pathogenic infections, and thus affect survival of pathogenic microorganisms. The compounds of the invention can therefore inactivate or inhibit essential survival processes of pathogenic microorganisms, which means that the compounds of the invention have the ability to decrease, suppress, attenuate, diminish, or arrest the development or progression of a pathogen-mediated infectious diseases in a subject.

Indeed, besides the presence of S1PL in humans, S1PL is also present in parasites, such as *Leishmania spp*. or *Schistosoma spp*., in bacteria, such as *Burkeholderia pseudomallei* or *Legionella pneumophilia*, as well as in pathogenic fungi and therefore may be targeted (inhibited) by the compounds of the present invention.

Infectious diseases are caused by pathogenic microorganisms selected from the group comprising bacteria, viruses, fungi and parasites. They are contagious and transmitted by insects, animals and/or by consuming contaminated food and/or water.

The treatment strategy therefore includes treatment with the compounds of the invention (as S1PL inhibitors) through the oral, topical and parenteral route, ideally for a short course after the onset of disease symptoms.

Thus another aspect of the present invention provides a method of preventing and/or treating infectious diseases in a subject, comprising administering a therapeutically or prophylactically effective amount of a compound of the invention to the subject, wherein the infectious diseases are caused by pathogenic microorganisms selected from the group comprising bacteria, viruses, fungi and parasites (including protozoa and helminths).

In a specific embodiment, the present invention provides a compound of the invention for use in a method for treating and/or preventing infectious diseases in a subject, wherein the infectious diseases are caused by pathogenic microorganisms selected from the group comprising bacteria, viruses, fungi and parasites (including protozoa and helminths). In another specific embodiment, the present invention provides a compound of the invention for use in a method of treating infectious diseases in a subject, wherein the infectious diseases are caused by pathogenic microorganisms selected from the group comprising bacteria, viruses, fungi and parasites (including protozoa and helminths).

The amount of the compound of the invention, dosing schedule, and route of administration will vary depending on the drug, the patient and the pathogenic microorganism, and can readily be determined by those of ordinary skill in the art.

In the context of the present invention, the subject may be selected from the group consisting of a mammal, a fish, a bird, and a crustacean. The mammal may be selected from the group consisting of a human, a bovine, an equine, a canine and an ungulate. The fish may be selected from the group consisting of a hagfish, a lamprey, a cartilaginous fish and a bony fish. The bird may be selected from the group consisting of chicken, a turkey, and a fowl. The crustacean may be selected from the group consisting of a shrimp, a crab, a lobster, a langouste.

In the context of the present invention, pathogenic microorganisms selected from the group comprising bacteria, viruses, fungi and parasites (including helminths and protozoa). The pathogenic microorganisms can have functional S1PL or not.

According to some preferred embodiments of the invention, pathogenic bacteria are selected from the group comprised of the following genuses of *Mycobacterium*, *Listeria*, *Legionella.*, *Burkholderia*, *Escherichia*, *Brucella*, *Bordatella*, *Helicobacter*, *Yersinia, Streptococcus*, *Staphylococcus*, *Clostridium*, *Pseudomonas*, *Diptheria*, *Klebsiella*, *Campylobacter*, *Acetinobacter*, *Cryptococcus*, *Clostridioides*, *Enterococcus*, *Shigella*, *Neisseria*, *Salmonella*, *Treponema*, *Cryptosporidium and Wolbachia*

According to some preferred embodiments of the invention, pathogenic fungi are selected from the group comprised of the following genuses of *Candida*, *Aspergillus*, *Fusarium*, *Cryptococcus*

According to some preferred embodiments of the invention, parasites including helminths are selected from the group comprising of the following genuses of *Leishmania*, *Trypanosoma*, *Schistosoma*, *Taenia*, *Echinococcus*, *Toxoplasma*, *Wuchereria*, *Brugia*, *Fasciola*, *Onchocerca*, *Dracunculus*, and *Plasmodium.*

According to some preferred embodiments of the invention, viruses are selected from the group comprising
- single-stranded positive sense RNA viruses, such as Flaviridae, Hepeviridae, Picornaviridae, Togaviridae
- single-stranded negative sense RNA viruses, such as Filoviridae, Paramyxoviridae, Rhabdoviridae
- double stranded RNA viruses, such as Reoviridae.
- double stranded DNA viruses, such as Herpesviridae, and Poxviridae.

The methods and compounds of the invention are useful in treating infectious diseases, for example preventing, inhibiting and/or ameliorating symptom of an infectious disease.

According to some embodiments of the invention, one or more active agents in addition to a compound of the invention can be used in methods of preventing and/or treating infectious diseases. The one or more additional active agents selected can be selected from the group comprising antibiotics, anti-bacterial agents, antifungal agents, antiparasitic agents, osmotic diuretics, anti-convulsants, anti-pyretics, immunomodulating drugs (including: anti-oxidants, anti- inflammatory drugs, immunosuppressants and immunostimulatory drugs), anti-viral agents and combinations thereof.

Examples of such additional active agents include:
- antibiotics and anti-bacterial agents, which include chemical agents that are active against bacteria. In common usage, an antibiotic is a substance or compound that kills or inhibits the growth of bacteria. Anti-bacterial antibiotics can be categorized based on their target specificity: "narrow-spectrum" antibiotics target particular types of bacteria, such as Gram-negative or Gram-positive bacteria, while broad-spectrum antibiotics affect a wide range of bacteria. Antibiotics which target the bacterial cell wall (such as penicillins, cephalosporins, cephems), or cell membrane (such as polymixins), or interfere with essential bacterial enzymes (such as quinolones, sulfonamides) usually are bactericidal in nature. Those which target protein synthesis such as the aminoglycosides, macrolides and tetracyclines are usually bacteriostatic. Three newer classes of antibiotics include: cyclic lipopeptides (such as daptomycin), glycylcyclines (such as tigecycline), and oxazolidinones (such as linezolid). Tigecycline is a broad-spectrum antibiotic, while the two others are useful for Gram-positive infections.
- antifungal agents, which include therapeutic compounds or bioactive agents that may be used to treat a fungal infection in a subject. An antifungal drug is a medication used to inhibit the growth of, or destroy fungi. Antifungal agents include, for example, polyene antifungals, imidazole, triazole and thiazole antifungals, allylamines, echinocandins, griseofulvin, flycystosine, undecylenic acid, among others.
- antiparasitic agents, which include therapeutic compounds or bioactive agents that are used to treat parasitic diseases including nematodes, cestodes, trematodes, infectious protozoa, and amoebas. Exemplary antiparasitic agents include: anti-nematodes (such as mebendazole, pyrantel pamoate, thiabendazole, diethycarbazine), anti-cestodes (such as niclosamide, praziquantel), anti-trematodes (such as praziquantel), anti-amoebics (such as rifampin and amphotericin B), anti-protozoals (such as melarsoprol, eflornithine, metronidazole, miltephosine, glucantime, paromycin sulfate,, Artimisinin, pentavalent antimonials, pentamidine, nifurtimox, Amphotericin B and tinidazole (and other nitroimidazole-based drugs), among others.
- anti-viral agents that includes virus adsorption inhibitors (e.g.: polysulphates, polysulphonates, polycarboxylates, polyoxometalates, zintevir, cosalane derivitives, Tromantine,), Entry and fusion inhibitors (e.g.: enfuvir, docosanol, enfuvirtide, Maraviroc, Pleconaril, Vicriviroc), Uncoating inhibitors (e.g.: Pleconaril, Amantadine, rimantadine), Viral synthesis inhibitors (including those that affect transcription, reverse transcription, integration of viral genome into host cell genomes, protein synthesis and processing (e.g.: Zidovudin, Lamivudine, Zalcitabine, Nevirapine, Efavirenz, Delavirdines, Saquinavir, Ininavir, fomivirsen, adefovir, atazanavir, mozenavir, tipranavir, abacavir, Acyclovir, Amprenavir,Cidovir, Darunavir, Didanosine Edoxudine,efavirenz,Emtricitabine, Entevavir, Famciclovir, Fosamprenavir, Methisazone,Nelfinavir, Norvir, Nevirapine, Penciclovir, Podophyllotoxin, Raltegravir, Ribavirin, Rimantadine, Ritonavir, Saquinavir, Sofobuvir, Stavudine, Trifluridine,Valaciclovir, ganciclovir, valganciclovir, Vidarabine, Ribavirin, Zalcitabine, azidothymidine)), Viral assembly (e.g.: rifampicin, atazanavir, Fosfonet, Indinavir, Lopinavir, Loviride, tipranavir), Viral release (e.g.: zanamivir, oseltamivir, RWJ270201, Mycophenolic acid, EICAR, VX487), among others.
- osmotic diuretics (such as mannitol and urea),
- anti-convulsants (such as diazepam, phenytoin, phenobarbital, and phenobarbitone),
- anti-pyretics (such as paracetamol),
- Immuno-modulating drugs including anti-oxidants, anti- inflammatory drugs (such as NSAIDS, steroids, cyclosporin, thalidomide, revlimid, anti-TNF antibodies (e.g., infliximab, etanercept), and pentoxifylline), immuno-stimulants (such as Toll-like receptor ligands (e.g.: Imiquimod), immune Receptor agonists, recombinant chemokines and/or cytokines (e.g.: recombinant TNF).

Thus in some embodiments of the invention, a compound of the invention is administered adjunctively with one or more additional active agents. Administration of a compound of the invention and one or more additional active agents may be concurrent (in the same dosage form or in separate dosage forms administered to the patient at approximately the same time), or not.

As described above, some embodiments include co-administering a compound of the invention and one or more additional active agent. By "co-administration" or "co-administering", it is meant that a compound of the invention and the one or more additional active agent are administered in such a manner that administration of a compound of the invention has an effect on the efficacy of the treatment of the one or more additional active agent, regardless of when or how they are actually administered. Thus in one embodiment, a compound of the invention and the one or more additional active agent are administered simultaneously. In one such embodiment, administration in combination is accomplished by combining a compound of the invention and the one or more additional active agent in a single dosage form. In another embodiment, a compound of the invention and the one or more additional active agent are administered sequentially. In one embodiment a compound of the invention and the one or more additional active agent are administered through the same route, such as orally. In another embodiment, a compound of the invention and the one or more additional active agent are administered through different routes, such as one being administered orally and another being administered parenterally and/or topically. In some embodiments, the time period between administration of a compound of the invention and administration of the co-administered one or more additional active agent can be about 1 hour, 2 hours, 3 hours, 5 hours, 8 hours, 10 hours, 12 hours, 15 hours, 1 8 hours, 20 hours, 24 hours, 36 hours, 48 hours, 3 days, 4 days, 5 days, 6 days, 7 days, 10 days, 14 days, 21 days, 28 days, or 30 days.

Another aspect of the invention provides a use of the compound of the invention for inhibiting SIP lyase in pathogens and parasites.

Another aspect of the present invention provides the pharmaceutical composition of the invention for use in a method of preventing and/or treating infectious diseases. In a specific embodiment, the present invention provides the pharmaceutical composition of the invention for use in a method for treating infectious diseases. In another specific embodiment, the present invention provides the pharmaceutical composition of the invention for use in a method of preventing infectious diseases.

Contrary to the prior art evidence that SIP pathway inhibition leads to favorable regulation of key cellular processes that contribute to tumor initiation and progression to cancer, the Applicant observed that gemcitabine-treated patients with elevated levels of plasmatic SIP concentrations, showed better overall survival rates. The activation of the SIP receptor 1 by SIP enhances the formation of adherent junctions, inhibits vascular endothelial growth factor signaling, suppresses sprouting and stabilizes new vascular connections.

The method according to the invention pertains to administrating a sphingosine-1-phosphate lyase inhibitor of the present invention as adjuvant or neoadjuvant therapy, i.e. during or prior to chemotherapy and/or radiotherapy. The neoadjuvant therapy according to the invention suppresses the sprouting angiogenesis and stabilizes the blood vessels. Consequently, the following chemotherapeutic agent has a better access to the tumor cells and therefore, an enhanced efficacy in its therapeutic effect. Likewise, the stabilization of the neovascular network improves the efficiency of a sequential anticancer radiotherapy.

Thus an aspect of the present invention provides a method for improving the efficacy of chemotherapy and/or radiotherapy, comprising administering to a subject in need thereof a therapeutically effective amount of the compound of the invention prior to the chemotherapy and/or radiotherapy.

In a specific embodiment, the present invention provides the compound of the invention for use in a method for improving the efficacy of a chemotherapy and/or a radiotherapy, wherein a therapeutically effective amount of the compound of the invention is administered to a subject prior to the chemotherapy and/or the radiotherapy.

Another aspect of the present invention provides a method of treatment of cancer comprising administering to a subject in need thereof a therapeutically effective amount of the compound of the invention prior to the chemotherapy and/or radiotherapy.

In a specific embodiment, the present invention provides the compound of the invention for use in a method of treatment of cancer, wherein a therapeutically effective amount of the compound of the invention is administered to a subject prior to the chemotherapy and/or radiotherapy.

In the context of the present invention, the chemotherapy can be the cytotoxic or the cytostatic chemotherapy that is selected from the group consisting of treatment with alkylating agents, topoisomerase inhibitors type 1 and type 2, antimetabolites, tubulin inhibitors, platinoids, and taxanes.

In the context of the present invention, the cancer is a solid tumor cancer displaying an abnormal tumor vasculature including but not limited to pancreatic adenocarcinoma, lung cancer, breast cancer, prostate cancer, cervix squamous cells carcinoma.

The sequential chemotherapy according to the invention involves a single anti-cancer agent or a combination of anti-cancer agents. Preferably, none of these agents displays anti-angiogenic properties. In one embodiment, the sequential chemotherapy involves agents selected from a group consisting of gemcitabine, paclitaxel, cisplatin and oxaliplatin.

In one embodiment, the subject displays a sphingosine-1-phosphate plasmatic concentration less than the median sphingosine-1-phosphate plasmatic concentration value of healthy volunteers. Since the sphingosine-1-phosphate plasmatic concentration measurement is analytical-method-dependent, the plasmatic concentration is measured with the same experimental protocol in the subject and in the healthy volunteers. In one embodiment, the subject displays a sphingosine-1-phosphate plasmatic concentration inferior to 700 nM, preferably inferior to 650 nM.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended claims, and all changes which come within the meaning and range of equivalency are intended to be embraced therein.

The foregoing description will be more fully understood with reference to the following Examples. Such Examples, are, however, exemplary of methods of practising the present invention and are not intended to limit the application and the scope of the invention.

### EXAMPLES

The evaluation of the thermodynamic binding parameters of different sphingosine-1-phosphate inhibitors has been carried out. The assay has been done with the phosphorylated compounds which are the actual S1PL inhibitors in-vivo.

Crystal structure of S1PL in the PDB (4Q6R) was used to perform the docking experiments. Docking was made with GOLD (Protein-Ligand docking using Genetic Algorithm) using the CHEMPLP scoring function (Protein-Ligand ANT System).

Poses were optimized and rescored with ChemScore to obtain ΔG binding energies. CHARMM force field was used to estimate the total binding and interaction energies of the rescored poses.

### Example 1:

### Phosphorylated THI metabolite and derivatives

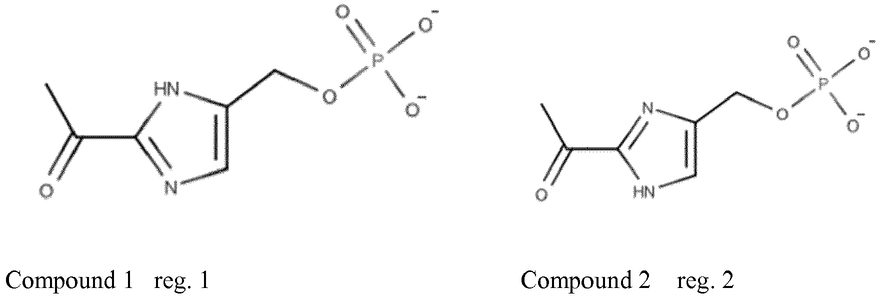

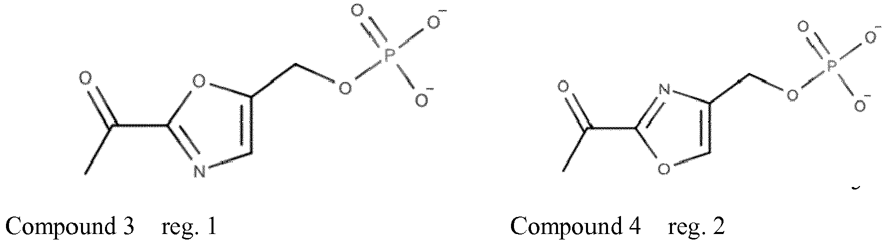

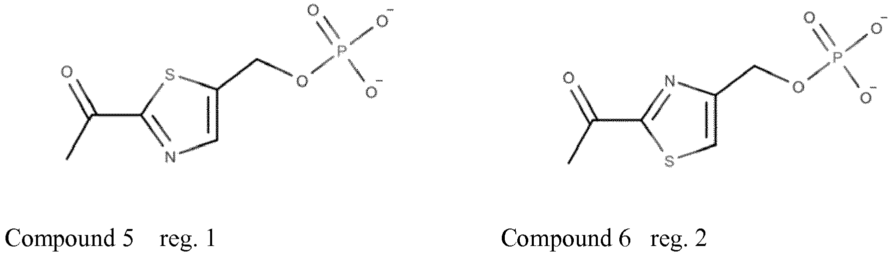

**Table 2: Thermodynamic parameters describing the binding of human sphingosine-1-phosphate Lyase inhibitors to the catalytic site of the enzyme.**

| Compounds | Total Interaction Energy Kcal/mol | Total VDW Interaction energy Kcal/mol | Total Electrostatic Interaction Energy Kcal/mol | ΔG Binging Energy Kcal/mol | Total Binging Energy Kcal/mol CHARMM* |
|---|---|---|---|---|---|
| S1P | -276.01532 | 9.39604 | -285.41136 | -31.254 | -504.836 |
| Compound 1 | -284.26823 | -5.66954 | -278.59869 | -28.13 | -188.55 |
| Compound 2 | -291.67197 | -2.81322 | -288.85875 | -28.05 | -186.28 |
| Compound 3 | -278.17480 | 3.30884 | -281.48364 | -28.09 | -706.22 |
| Compound 4 | -237,43263 | -0.6689 | -236.76875 | -15.10 | -695.54 |
| Compound 5 | -108.66511 | 177.49551 | -286.16062 | -30.55 | -710.18 |
| Compound 6 | -227.89978 | -4.27660 | -223.62118 | -11.99 | -701.26 |

| | | | | | |
|---|---|---|---|---|---|
| S1P: Sphingosine-1-phosphate. VDW: Van Der Walls. *Absolute binding free energy | | | | | |

**Sphingosine-1-Phosphate (S1P):** SIP displays a high binding energy mainly coming from electrostatic interaction. A slight energetic penalty comes from the Van de Walls repulsion of about 9 Kcal/mol.
**Phosphorylated compounds 1 and 2 (Tautomer 1 and 2):** Tautomers correspond to the two possible position of the basic nitrogen of the imidazole ring. The phosphorylated forms of the compounds 1 and 2 display a strong binding energy mainly due to electrostatic interaction. The position of the nitrogen of the imidazole ring has a marginal impact on the binding. For both tautomers, the total interaction energy is close to the one of S1P. However, the total binding energy as calculated with all thermodynamics parameters provided by the force field CHARMM is much lower than the one of S1P (188 vs 504).
**Phosphorylated compounds 3 and 4 (oxazole ring regioisomers 1 and 2):** These compounds display a high binding energy mainly due to electrostatic interaction. The values of the total interaction energy and ΔG are close to those observed with the imidazole ring (THI). However, the total binding energy as calculated by CHARMM is much higher than the one observed for the phosphorylated compounds 3 and 4 and higher than the one of S1P (-706 vs -504).
**Phosphorylated compounds 5 and 6 (thiazole ring regioisomer 1 and 2):** These compounds displays a high binding energy mainly due to electrostatic interaction. The values of the total interaction energy is markedly lower than those of the imidazole and oxazole containing molecules, due a marked energetic penalty (VdW: +177). ΔG value is close to those observed with the imidazole ring and oxazole containing molecule. However, as seen with the oxazole molecule, the total binding energy as calculated by CHARMM is much higher than the one observed for the phosphorylated compounds 1 and 2 and higher than the one of S1P (-710 vs 504).

### Example 2:

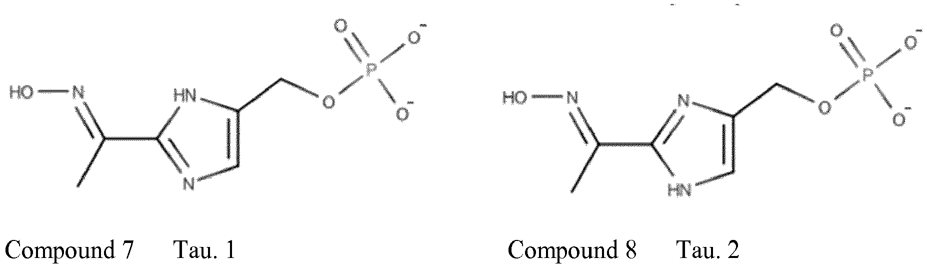

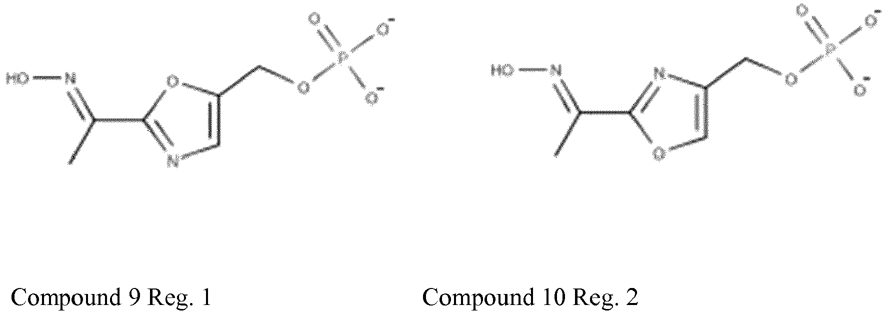

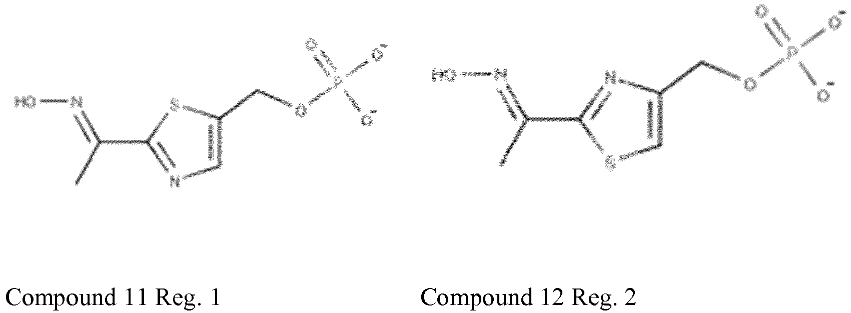

**Table 3: Thermodynamic parameters describing the binding of human sphingosine-1-phosphate Lyase inhibitors LX2931 and derivatives to the catalytic site of the enzyme.**

| Compounds | Total Interaction Energy Kcal/mol | Total VDW Interaction energy Kcal/mol | Total Electrostatic Interaction Energy Kcal/mol | ΔG Binging Energy Kcal/mol | Total Binging Energy Kcal/mol CHARMM* |
|---|---|---|---|---|---|
| SIP | -276.01532 | 9.39604 | -285.41136 | -31.25 | -504.836 |
| Compound 7 | -267.35952 | 5.62673 | -272.98626 | -27.92 | -192.63 |
| Compound 8 | -247.50954 | 28.09285 | -275.60239 | -27.10 | -186.75 |
| Compound 9 | -311.66049 | -3.49726 | -308.16333 | -15.45 | -717.80 |
| Compound 10 | -234.22074 | -5.24975 | -228.97099 | -17.01 | -725.34 |
| Compound 11 | -255.08918 | -6.47355 | -248.61563 | -18.93 | -725.34 |
| Compound 12 | -327.26240 | -3.97969 | -323.28271 | -15.55 | -717.42 |

| | | | | | |
|---|---|---|---|---|---|
| S1P: Sphingosine-1-phosphate. VDW: Van Der Walls. *Absolute binding free energy | | | | | |

**Phosphorylated compounds 7 and 8 (Tautomer 1 and 2):** Tautomers correspond to the two possible position of the basic nitrogen of the imidazole ring. The phosphorylated forms of these compounds display a strong binding energy mainly due to electrostatic interaction. For studied tautomers, the total interaction energy is close to the one of S1P. However, the total binding energy as calculated with all thermodynamics parameters provided by the force field CHARMM is much lower than the one of S1P (187 vs 504).
**Phosphorylated compounds 9 and 10 (oxazole ring regioisomer 1 and 2):** These compounds display as all phosphorylated compounds a high binding energy mainly due to electrostatic interaction. The total interaction energy values are close or higher to those observed with the imidazole ring. However, the total binding energy as calculated by CHARMM is much higher than the one observed for the phosphorylated imidazole containing compounds and higher than the one of S1P (-717 and -725 vs -504).
**Phosphorylated compounds 11 and 12 (thiazole ring regioisomer 1 and 2):** These compounds display a high binding energy mainly due to electrostatic interaction. The values of the total interaction energy is markedly are close to those observed with the oxazole containing compounds. ΔG value is close to those observed with oxazole containing compounds but lower compared to those displaying by the imidazole ring. However, as seen with the oxazole containing compounds, the total binding energy as calculated by CHARMM is much higher than the one observed for the phosphorylated imidazole containing compounds and higher than the one of S1P (-725 and -717 vs 504)

### Example 3:

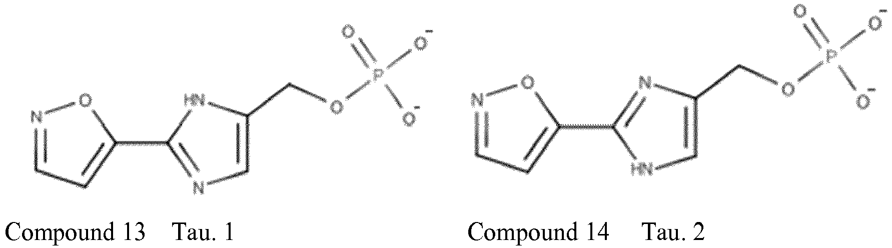

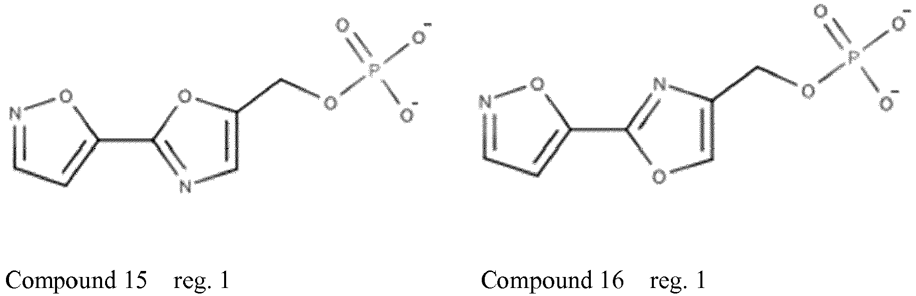

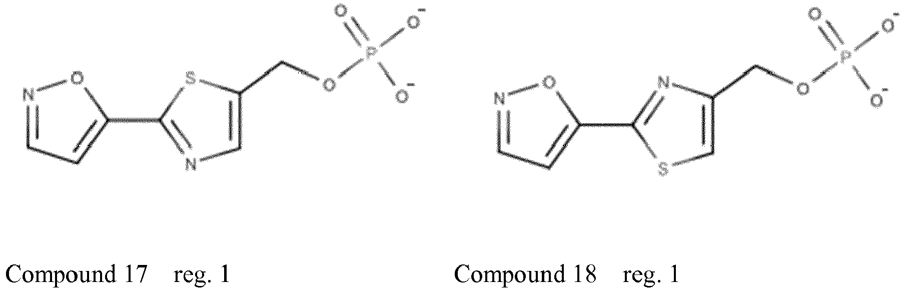

**Table 4: Thermodynamic parameters describing the binding of human sphingosine-1-phosphate Lyase inhibitors LX2932 and derivatives to the catalytic site of the enzyme.**

| Compounds | Total Interaction Energy Kcal/mol | Total VDW Interaction energy Kcal/mol | Total Electrostatic Interaction Energy Kcal/mol | ΔG Binging Energy Kcal/mol | Total Binging Energy Kcal/mol CHARMM* |
|---|---|---|---|---|---|
| S1P | -276.01532 | 9.39604 | -285.41136 | -31.25 | -504.836 |
| Compound 13 | -229.67669 | -4.61683 | -225.05986 | -23.21 | -754.60 |
| Compound 14 | -295.00186 | -1.84194 | -293.15992 | -23.59 | -739.17 |
| Compound 15 | -235.52535 | 7.53849 | -243.06384 | -18.14 | -697.66 |
| Compound 16 | -240.39398 | -7.74407 | -232.64991 | -17.90 | -702.19 |
| Compound 17 | -229.90385 | -5.49813 | -224.40571 | -16.78 | -696.31 |
| Compound 18 | -317.02957 | -4.30811 | -312.72146 | -21.34 | -712.67 |

| | | | | | |
|---|---|---|---|---|---|
| S1P: Sphingosine-1-phosphate. VDW: Van Der Walls. *Absolute binding free energy | | | | | |

**Phosphorylated compounds 13 and 14 (Tautomer 1 and 2):** Tautomers correspond to the two possible position of the basic nitrogen of the imidazole ring. The phosphorylated forms of these compounds display a strong binding energy mainly due to electrostatic interaction. For studied tautomers, the total interaction energy is close to the one of S1P. In contrast with the other imidazole containing ring, the total binding energy as calculated with all thermodynamics parameters provided by the force field CHARMM are extremely high (-754 and -730 for tautomer one, compound 13 and tautomer two, compound 14 respectively).
**Phosphorylated compound 15 and 16 (regioisomer 1 and 2):** These compounds display as all phosphorylated compounds a high binding energy mainly due to electrostatic interaction. The total interaction energy values are close or higher to those observed that contain the imidazole ring. The total binding energy as calculated by CHARMM is slightly lower compared to the one observed for the phosphorylated imidazole containing compound and higher than the one of S1P (-697 and -702 vs -504).
**Phosphorylated compounds 17 and 18 (regioisomer 1 and 2):** These compounds display a high binding energy mainly due to electrostatic interaction. The value of the total interaction energy of compound 17 is close to those observed with the oxazole containing compounds. ΔG value is close as well to those observed with oxazole containing compounds but lower compared to those displaying by the imidazole ring. Interestingly, compound 18 displays a high interaction energy (-317), higher than the one observed for all investigated compounds. Compound 18 displays also a high ΔG binding energy as well as a high total binding energy as calculated by CHARMM (713).

### Conclusion:

Based on thermodynamics parameters it can be concluded:
1) Compounds 1 to 6: both oxazole and thiazole containing phosphorylated compounds have a higher S1PL inhibitory potency compared to the phosphorylated imidazole containing compounds.
2) Compounds 7 to 12: both oxazole and thiazole containing phosphorylated compounds have a higher S1PL inhibitory potency compared to the phosphorylated imidazole containing compounds.
3) Compounds 13 to 18: both oxazole and thiazole containing phosphorylated compounds have a similar S1PL inhibitory potency compared to the phosphorylated imidazole containing compounds. The thiazole derivative compound 18 displays the best binding parameters compared to the compounds 15, 16 and 17.

## Claims

1. A compound of Formula I 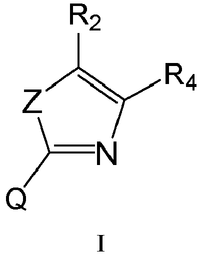 wherein
Z is selected from the group comprising O, S, NH;
Q is 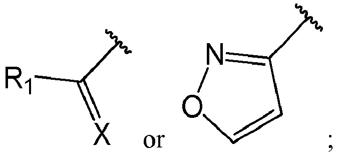
X is O or NR₃;
R₁ is selected from the group comprising OR_{A}, NHOH, hydrogen, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl;
R₂ is selected from the group comprising OR_{B}, C(O)OR_{B}, hydrogen, halogen, nitrile, optionally substituted hydroxyalkyl, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl;
R₃ is selected from the group comprising OR_{C}, N(R_{C})₂, NHC(O)R_{C}, NHSO₂R_{C}, hydrogen;
R₄ is selected from the group comprising OR_{D}, OC(O)R_{D}, N(R_{E})₂, hydrogen, halogen, optionally substituted hydroxyalkyl, optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl; and
each of R_{A}, R_{B}, R_{C}, R_{D}, and R_{E} is independently hydrogen or optionally substituted alkyl, optionally substituted aryl, optionally substituted alkylaryl, optionally substituted arylalkyl, optionally substituted heteroalkyl, optionally substituted heterocycle, optionally substituted alkylheterocycle, optionally substituted heterocyclealkyl.

2. The compound of claim 1, wherein Z is O or S.

3. The compound of any one of claims 1-2, wherein R₁ is C₁-C₅ alkyl.

4. The compound of any one of claims 1-3, wherein X is O or N-OH.

5. The compound of any one of claims 1-4, wherein R₂ is hydrogen and R₄ is -CH₂-OH, or wherein R₂ is -CH₂-OH and R₄ is hydrogen.

6. The compound of claim 1, selected from the group comprising

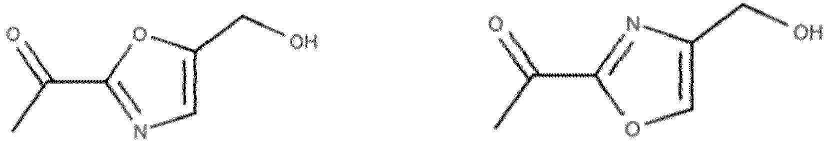

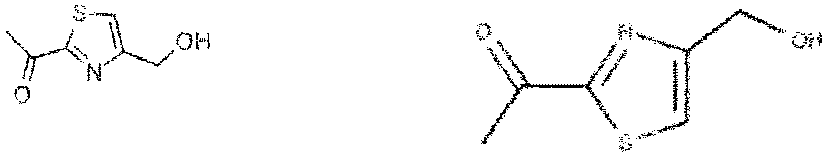

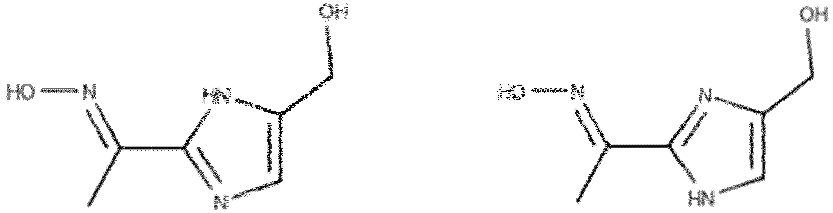

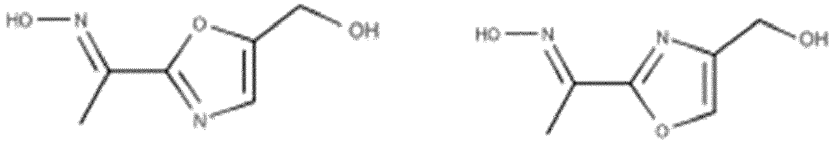

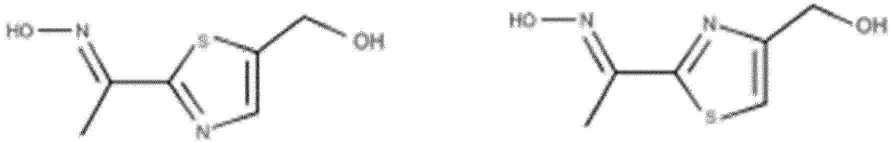

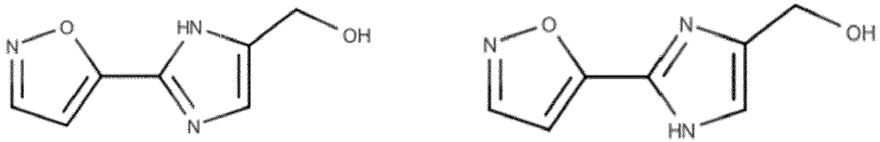

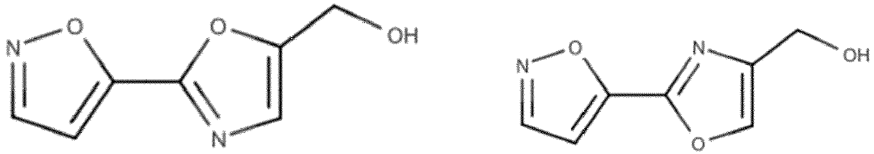

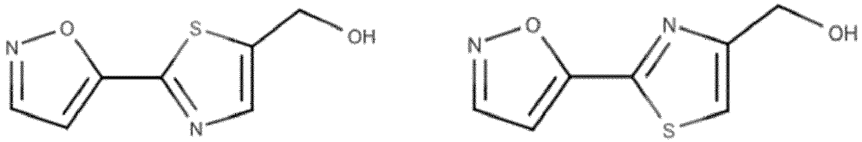

7. The compound of claim 1, selected from the group comprising

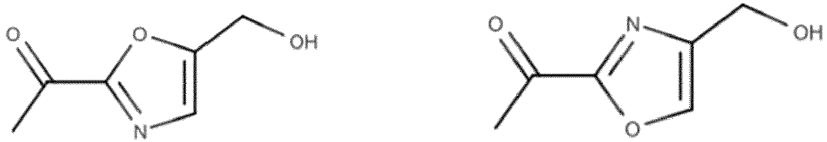

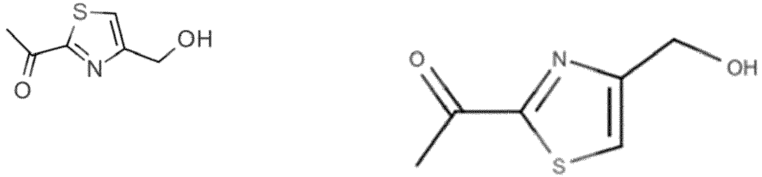

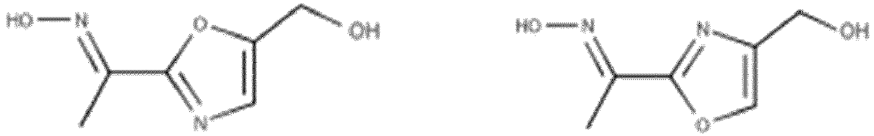

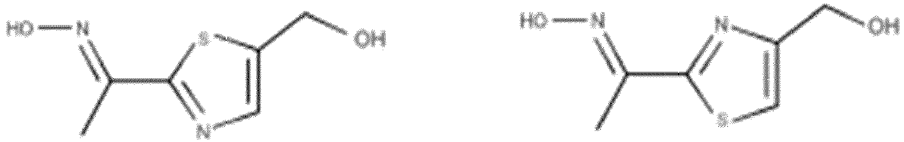

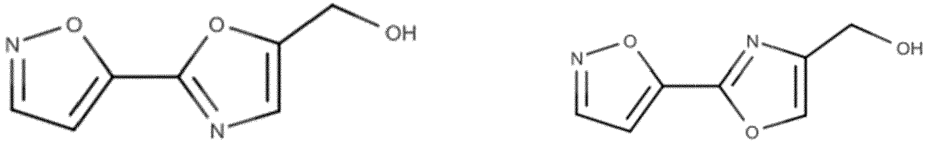

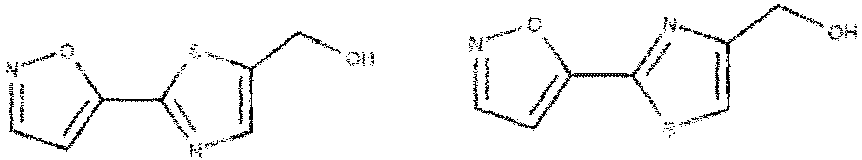

8. A pharmaceutical composition comprising the compound of any one of claims 1-7 and pharmaceutically acceptable excipients and/or carriers.

9. The compound of any one of claims 1-7 for use in a method of increasing the amount of SIP in a subject.

10. The compound of any one claims 1-7 for use in a method of preventing and/or treating infectious diseases in a subject, wherein the infectious diseases are caused by pathogenic microorganisms selected from the group comprising bacteria, viruses, fungi and parasites.

11. The compound for use according to claim 10, wherein the method further comprising administering to the subject one or more additional active agents selected from the group comprising antibiotics, anti-bacterial agents, antifungal agents, antiparasitic agents, osmotic diuretics, anti-convulsants, anti-pyretics, immunomodulating drugs (including: anti-oxidants, anti- inflammatory drugs, immunosuppressants and immunostimulatory drugs), anti-viral agents and combinations thereof.

12. The compound for use according to any one of claims 9-11, wherein the subject is selected from the group consisting of a mammal, a fish, a bird, and a crustacean.

13. The compound of any one of claims 1-7 for use in a method for improving the efficacy of a chemotherapy and/or a radiotherapy, wherein a therapeutically effective amount of the compound of any one of claims 1-7 is administered to a subject prior to or during the chemotherapy and/or the radiotherapy.

14. The compound of any one of claims 1-7 for use in a method of treatment of cancer, wherein a therapeutically effective amount of the compound of any one of claims 1-7 is administered to a subject prior to or during the chemotherapy and/or the radiotherapy.
